# EUROPÄISCHE PATENTSCHRIFT

(11) **EP 1 476 436 B1**
(45) Veröffentlichungstag und Bekanntmachung des Hinweises auf die Patenterteilung: **29.06.2011**
(21) Anmeldenummer: 03706553.9
(22) Anmeldetag: 21.02.2003
(51) Int. Cl.: C07D 239/42

(54) **VERFAHREN ZUR HERSTELLUNG VON 2-ANILINO-4,6-DIMETHYLPYRIMIDIN**
METHOD FOR PRODUCING 2-ANILINO-4,6-DIMETHYLPYRIMIDINE
PROCEDE DE PRODUCTION DE 2-ANILINO-4,6-DIMETHYLPYRIMIDINE

(30) Priorität: 22.02.2002 DE 10207376
(43) Veröffentlichungstag der Anmeldung: 17.11.2004
(73) Patentinhaber: AlzChem Trostberg GmbH, 83308 Trostberg (DE)
(72) Erfinder: GASTNER, Thomas, 83308 Trostberg (DE); HÖLZL, Anita, 84574 Taufkirchen (DE); HUBER, Claudia, 83352 Altenmarkt (DE); MASCHA, Alfred, 84518 Garching (DE)
(74) Vertreter: Dey, Michael
(86) Internationale Anmeldenummer: PCT/EP2003/001808
(87) Internationale Veröffentlichungsnummer: WO 2003/070708

(56) Entgegenhaltungen:
- EP-A- 0 717 038
- KREUTZBERGER A ET AL: "ANTIBAKTERIELLE WIRKSTOFFE, 10.MITT. 2-(METHYLTHIOANILINO) PYRIMIDINE. ANTIBACTERIAL DRUGS, X: 2-(METHYLTHIOANILINO) PYRIMIDINES" ARCHIV DER PHARMAZIE, VCH VERLAGSGESELLSCHAFT MBH, WEINHEIM, DE, Bd. 318, 1985, Seiten 1043-1045, XP002043905 ISSN: 0365-6233
- ARVANITIS A G ET AL: "NON-PEPTIDE CORTICOTROPIN-RELEASING HORMONE ANTAGONISTS: SYNTHESES AND STRUCTURE-ACTIVITY RELATIONSHIPS OF 2-ANILINOPYRIMIDINES AND -TRIAZINES" JOURNAL OF MEDICINAL CHEMISTRY, AMERICAN CHEMICAL SOCIETY. WASHINGTON, US, Bd. 42, Nr. 5, 1999, Seiten 805-818, XP001068996 ISSN: 0022-2623

## Beschreibung

Gegenstand der vorliegenden Erfindung ist ein Verfahren zur Herstellung von 2-Anilino-4,6-dimethylpyrimidin, das auch als Pyrimethanil bezeichnet wird.

Pyrimethanil hat eine große Bedeutung als Pflanzenschutzmittel und wird als solches in verschiedenen Formulierungen und Wirkstoffkombinationen als Fungizid eingesetzt.

In der Literatur sind mehrere Verfahren zur Darstellung von 2-Anilino-pyrimidin-Derivaten bekannt. Mit den zumeist älteren Verfahren wird die Zielverbindung jedoch unter Einsatz teurer Reagenzien in nur mäßigen Ausbeuten erhalten. Gemäß EP-B 224 339 wird z.B. Formaniliden mit 2-Methansulfonylpyrimidin-Derivaten umgesetzt; DD 151 404 empfiehlt die Verwendung von 2-Chlor-pyrimidin-Derivaten und Anilin.

In einem neueren Verfahren zur Darstellung von 2-Anilino-4,6-dimethylpyrimidin wird ein Phenylguanidin-Salz mit Acetylaceton ohne Lösemittel umgesetzt; wesentlich für das Gelingen der Reaktion ist dabei, dass neben entstehendem Kohlendioxid auch das entstehende Reaktionswasser während der Reaktion im Vakuum entfernt wird (EP-A 717 038). Das so erhaltene Produkt fällt als Schmelze an, woraus beim Abkühlen sehr grobe, beige Kristalle erhalten werden.

Insgesamt muss die Reproduzierbarkeit dieses Verfahrens aber als problematisch angesehen werden. So wurde der Gehalt an 2-Anilino-4,6-dimethylpyrimidin über HPLC-Flächenprozente ausgewertet. Mit dieser Methode können jedoch viele Nebenprodukte nicht erfasst werden, die aufgrund ihrer größeren Masse erst nach einer sehr langen Laufzeit im Chromatogramm auftreten. Erfolgt die Quantifizierung von 2-Anilino-4,6-dimethylpyrimidin hingegen über einen externen Standard, werden auch Anteile an nicht verdampfbaren Substanzen und insbesondere polare und hochmolekulare Verbindungen gefunden, die sich nachteilig auf die Reinheit des Produktes auswirken.

Aus den geschilderten Nachteilen des Standes der Technik hat sich für die vorliegende Erfindung somit die Aufgabe gestellt, ein Verfahren zur Herstellung von 2-Anilino-4,6-dimethylpyrimidin bereitzustellen, bei dem Phenylguanidin und/oder eines seiner Salze mit Acetylaceton umgesetzt wird und mit dem die Verbindung in guten Ausbeuten und Reinheiten als feinkristallines Pulver erhalten wird. Dabei sollte nach Möglichkeit auf die Verwendung von teuren und umweltbedenklichen Stoffen verzichtet werden.

Diese Aufgabe wird gelöst, indem man die Umsetzung unter Zugabe von Wasser in einer Menge von 5-500 % der Masse an eingesetzter Phenylguanidin-Komponente durchführt und das bei der Reaktion entstehende Wasser nicht abtrennt.

Überraschenderweise und im Gegensatz zur vorherrschenden Meinung gemäß Stand der Technik hat sich gezeigt, dass es gerade in Gegenwart von Wasser möglich ist, das 2-Anilino-4,6-dimethylpyrimidin in hohen Ausbeuten und mit höchster Reinheit in feinkristalliner Form zu erhalten. Auf diese Weise erhält man in technisch einfacher Weise bei umweltschonenden Bedingungen ein feinkristallines Produkt mit bester Reinheit und in sehr guten Ausbeuten.

Als Phenylguanidin-Komponente werden gemäß vorliegender Erfindung bevorzugt Phenylguanidinhydrogencarbonat und/oder Phenylguanidincarbonat eingesetzt, deren Herstellung aus der Literatur gut bekannt ist (vgl. Firmenbroschüre der SKW Trostberg AG, S. 97 (1978); EP-A 560 726).

Bei der Zugabereihenfolge ist es entweder möglich, die Phenylguanidin-Komponente in Wasser vorzulegen und anschließend das Acetylaceton zuzutropfen oder aber bei umgekehrter Reihenfolge die Phenylguanidin-Komponente zu einer Mischung aus Wasser und Acetylaceton zu geben. Dabei ist die Zugabegeschwindigkeit in der Regel in einem weiten Bereich variierbar und wird nur durch die Abführbarkeit des bei der Reaktion entstehenden Kohlendioxids begrenzt. Möglich ist aber auch ein gleichzeitiges Vorlegen aller drei Komponenten.

Erfindungsgemäß wird der Reaktion zusätzlich Wasser hinzugefügt, erfindungsgemäß mindestens 5 % der Masse, bevorzugt mindestens 10 % der Masse der Phenylguanidinkomponente. Besonders bevorzugt beträgt die zugesetzte Wassermenge vor dem Kristallisieren zwischen 50 und 150 % der Masse der Phenylguanidin-Komponente.

Es ist im Rahmen der Erfindung als bevorzugt anzusehen, die Phenylguanidin-Komponente in einem Molverhältnis zum Acetylaceton einzusetzen, das 1 : 0,8 bis 2,0 und insbesondere 1 : 1,0 bis 1,1 beträgt. Das jeweilige Phenylguanidinsalz kann hierbei erfindungsgemäß auch in wasserfeuchter Form verwendet werden, wodurch eine vorherige Trocknung in vorteilhafter Weise entfallen kann.

Die Umsetzung kann ferner gemäß vorliegender Erfindung bei einem Druck zwischen 0,1 und 10 bar und im breiten Temperaturbereich von 40 bis 180 °C durchgeführt werden, wobei Drücke zwischen 1 und 5 bar und insbesondere Atmosphärendruck sowie Temperaturen zwischen 80 bis 100 °C als besonders bevorzugt anzusehen sind. Bei diesen Temperatur- und Druckbereichen liegt die Reaktionszeit in Abhängigkeit von der Dosierzeit zwischen wenigen Minuten und 4 Stunden. Dabei haben längere Reaktionszeiten auf die Produktqualität und Ausbeute keinerlei negativen Einfluss.

Die Reaktionsmischung besteht am Ende der Reaktion aus zwei flüssigen Phasen, nämlich einer Wasser- und einer Produktphase. Das Produkt kann aus diesem System problemlos abgetrennt werden und erstarrt beim Erkalten. Besonders vorteilhaft ist es, das erhaltene Zweiphasensystem unter guter Durchmischung abzukühlen, wobei das Produkt in feinkristalliner Form anfällt. Auch die Abkühlrate ist nicht limitierend und kann in einem weiten Bereich variieren, wobei allerdings Temperaturschritte zwischen 10 und 50 °C pro Stunde empfohlen werden.

Die so erhaltenen Kristalle können in jeder geläufigen Art und Weise von der flüssigen Phase abgetrennt werden. Die Trocknung des Produktes erfolgt in der Regel bei Drücken zwischen 0,1 bis 1000 mbar und bei Temperaturen zwischen 30 bis 95 °C, wobei sich Drücke zwischen 10 bis 20 mbar und Temperaturen zwischen 40 bis 70 °C besonders empfehlen.

Insgesamt zeichnet sich das erfindungsgemäße Verfahren durch eine sehr einfache technische Durchführbarkeit und weiterhin durch eine hohe Umweltverträglichkeit aus. Die nach der Reaktion abgetrennte Mutterlauge kann außerdem als Lösemittel für weitere Umsetzungen recycliert werden, was die Wirtschaftlichkeit des vorgeschlagenen Verfahrens zusätzlich unterstreicht.

Evtl. im Überschuss eingesetzte Acetylaceton- und/oder Phenylguanidin-Mengen sind unter den gewählten Reaktionsbedingungen stabil und können nach der Reaktion nahezu vollständig aus der Mutterlauge abgetrennt und wiederverwendet werden. Weiterhin ist es problemlos möglich und sowohl energetisch als auch unter Umweltgesichtspunkten besonders günstig, die Mutterlauge mit dem enthaltenen Acetylaceton oder der Phenylguanidin-Komponente für weitere Umsetzungen zu verwenden.

Die nachfolgenden Beispiele veranschaulichen die mit dem erfindungsgemäßen Verfahren verbundenen Vorteile.

### Beispiele

### 1. Vergleichsbeispiel gemäß EP-A 717 038

Phenylguanidincarbonat (51,18 g, 146 mmol) und Acetylaceton (37,7 g, 377 mmol) wurden unter Rühren in einen Rundkolben mit angeschlossenem Wasserabscheider mit Rückflusskühler bei einem Vakuum von 260 mbar langsam erhitzt. Bei etwa 55 °C begann die Abscheidung einer wässrigen Phase und es entwickelte sich Kohlendioxid. Während die entstehenden Wasser- und Kohlendioxid-Mengen kontinuierlich abgetrennt werden, wurde die Temperatur innerhalb von 2 h auf 75 °C erhöht und gleichzeitig der Druck auf 190 mbar abgesenkt. Das anfangs inhomogene Gemisch wandelte sich dabei in eine orange-braune, klare und wasserfreie Flüssigphase um. Der Druck wurde dann auf 100 mbar abgesenkt und der Ansatz noch eine weitere Stunde bei 95 °C gerührt. Anschließend wurde das verbliebene Acetylaceton bei 10 mbar komplett entfernt. Die zurückgebliebene Masse wurde in eine Schale gegossen, wobei sich beim Abkühlen ein beigefarbener Feststoff bildete.

| | |
|---|---|
| Ausbeute: | 53,9 g 2-Anilino-4,6-dimethylpyrimidin (73 %ig GC, 66 % d. Th) |

### 2. Erfindungsbeispiele

2.1 In einem Kolben mit Rückflusskühler und Tropftrichter wurde Wasser (15 ml) vorgelegt und Phenylguanidincarbonat (34,1 g, 97,3 mmol) eingetragen. Der Ansatz wurde auf 85 °C erwärmt und langsam Acetylaceton (22,6 g, 225,8 mmol) zugetropft, wobei sich nach einiger Zeit Kohlendioxid bildete. Nach beendeter Kohlendioxid-Entwicklung wurde noch 3 Stunden bei 95 °C gerührt und dann langsam auf etwa 20 °C abgekühlt. Die Suspension wurde abgesaugt, das Produkt dreimal mit je 10 ml Wasser gewaschen und im Trockenschrank bei 60 °C getrocknet.

| | |
|---|---|
| Ausbeute: | 34,1 g 2-Anilino-4,6-dimethylpyrimidin (98,2 %ig GC, 87% d. Th) |

2.2 In einem Kolben mit Rückflusskühler und Tropftrichter wurden Wasser (10 ml) und Acetylaceton (45,2 g, 451,6 mmol) vorgelegt und dann bei 85 °C langsam Phenylguanidincarbonat (72,0 g, 205,2 mmol) eingetragen. Nach beendeter Kohlendioxid-Entwicklung wurde bei 95 °C 3 Stunden nachgerührt, dann Wasser (50 ml, 90 °C) zugegeben und langsam auf 20 °C abgekühlt. Die Mischung wurde abgesaugt, das Produkt dreimal mit je 10 ml Wasser (60 °C) gewaschen und im Trockenschrank bei 60 °C getrocknet.

| | |
|---|---|
| Ausbeute: | 74,4 g 2-Anilino-4,6-dimethylpyrimidin (99,1 %ig GC, 91 % d. Th) |

2.3 In einem Kolben mit Rückflusskühler und Tropftrichter wurden Wasser (10 ml) und Acetylaceton (55 g, 549 mmol) vorgelegt und dann bei 85 °C Phenylguanidinhydrogencarbonat (98,6 g, 500 mmol) langsam eingetragen. Nach beendeter Kohlendioxid-Entwicklung wurde bei 95 °C 3 Stunden nachgerührt und dann Wasser (70 ml, 85 °C) zugegeben. Die Emulsion wurde langsam auf 20 °C abgekühlt, dann abgesaugt, das Produkt dreimal mit je 10 ml Wasser (60 °C) gewaschen und im Trockenschrank bei 60°C getrocknet.

| | |
|---|---|
| Ausbeute: | 87,9 g 2-Anilino-4,6-dimethylpyrimidin (98,6 %ig GC, 87 % d. Th) |

## Patentansprüche

1. Verfahren zur Herstellung von 2-Anilino-4,6-dimethylpyrimidin durch Umsetzung von Phenylguanidin und/oder einem seiner Salze mit Acetylaceton,
**dadurch gekennzeichnet,**
**dass** man die Umsetzung unter Zugabe von Wasser in einer Menge von 5-500% der Masse an eingesetzter Phenylguanidin-Komponente durchführt und das bei der Reaktion entstehende Wasser nicht abtrennt.

2. Verfahren nach Anspruch 1,
**dadurch gekennzeichnet,**
**dass** man als Phenylguanidin-Salz Phenylguanidincarbonat und/oder Phenylguanidinhydrogencarbonat, bevorzugt in wasserfeuchter Form, einsetzt.

3. Verfahren nach einem der Ansprüche 1 oder 2,
**dadurch gekennzeichnet,**
**dass** man die Reaktion bei 0,1 bis 10 bar, vorzugsweise bei 1 bis 5 bar und insbesondere bei Atmosphärendruck, durchführt.

4. Verfahren nach einem der Ansprüche 1 bis 3,
**dadurch gekennzeichnet,**
**dass** man die Umsetzung im Temperaturbereich von 40 bis 180 °C, vorzugsweise bei 80 bis 100 °C, durchführt.

5. Verfahren nach einem der Ansprüche 1 bis 4,
**dadurch gekennzeichnet,**
**dass** das Molverhältnis der Phenylguanidin-Komponente zum Acetylaceton zwischen 1 : 0,8 bis 2,0 und bevorzugt zwischen 1 : 1,0 bis 1,1 liegt.

6. Verfahren nach einem der Ansprüche 1 bis 5,
**dadurch gekennzeichnet,**
**dass** man das Produkt in kristalliner Form isoliert, insbesondere durch Abkühlen aus dem bei der Reaktion gebildeten Zweiphasensystem.

## Claims

1. Method for producing 2-anilino-4,6-dimethylpyrimidine by reacting phenylguanidine and/or one of its salts with acetylacetone, **characterized in that**,
the reaction is carried out with the addition of water in an amount of 5 - 500 % of the mass of the phenylguanidine component that is used and the water formed during the reaction is not separated.

2. Method according to claim 1,
**characterized in that**,
phenylguanidine carbonate and/or phenylguanidine hydrogen carbonate preferably in a water-moist form is used as the phenylguanidine salt.

3. Method according to one of the claims 1 or 2,
**characterized in that**,
the reaction is carried out at 0.1 to 10 bar, preferably at 1 to 5 bar and in particular at atmospheric pressure.

4. Method according to one of the claims 1 to 3,
**characterized in that**,
the reaction is carried out in a temperature range of 40 to 180°C, preferably at 80 to 100°C.

5. Method according to one of the claims 1 to 4,
**characterized in that**,
the molar ratio of the phenylguanidine component to acetylacetone is between 1 : 0.8 to 2.0 and preferably between 1 : 1.0 to 1.1.

6. Method according to one of the claims 1 to 5, **characterized in that**,
the product is isolated in a crystalline form, in particular by cooling from the two-phase system formed in the reaction.

## Revendications

1. Procédé de production de la 2-anilino-4,6-diméthylpyrimidine par réaction de la phénylguanidine et/ou de l'un de ses sels avec l'acétylacétone, **caractérisé en ce que** l'on conduit la réaction en ajoutant de l'eau en une quantité de 5-500 % de la masse de composant phénylguanidine utilisé et l'on ne sépare pas l'eau formée pendant la réaction.

2. Procédé selon la revendication 1 **caractérisé en ce que** l'on utilise comme sel de phénylguanidine le carbonate de phénylguanidine et/ou l'hydrogénocarbonate de phénylguanidine, de préférence sous forme humide.

3. Procédé selon l'une des revendications 1 et 2 **caractérisé en ce que** l'on conduit la réaction à 0,1 à 10 bar, de préférence à 1 à 5 bar et en particulier à la pression atmosphérique.

4. Procédé selon l'une des revendications 1 à 3 **caractérisé en ce que** l'on conduit la réaction dans le domaine de température de 40 à 180°C, de préférence à 80 à 100°C.

5. Procédé selon l'une des revendications 1 à 4 **caractérisé en ce que** le rapport molaire du composant phénylguanidine à l'acétylacétone est situé entre 1 : 0,8 et 2,0 et de préférence entre 1 : 1,0 et 1,1.

6. Procédé selon l'une des revendications 1 à 5 **caractérisé en ce que** l'on isole le produit sous forme cristalline, en particulier par refroidissement à partir du système à deux phases formé pendant la réaction.
